# EUROPEAN PATENT APPLICATION

(11) **EP 3 346 275 A1**
(43) Date of publication of application: **11.07.2018**
(21) Application number: 16842148.5
(22) Date of filing: 16.08.2016
(51) Int. Cl.: G01N 35/00, G04G 5/00, B01L 3/00

(54) **TESTING APPARATUS AND CONTROL METHOD THEREFOR**

(30) Priority: 31.08.2015 KR 20150122596
(71) Applicant: Samsung Electronics Co., Ltd., Suwon-si, Gyeonggi-do 16677 (KR)
(72) Inventor: GWON, Se Do, Goyang-si Gyeonggi-do 10544 (KR)
(74) Representative: Gulde & Partner
(86) International application number: PCT/KR2016/008970
(87) International publication number: WO 2017/039182

(57) **Abstract**

A test apparatus and control method thereof are disclosed, which relate to a technology for providing an in-vitro diagnostic apparatus for enabling a user to change time information used in the system, and displaying the changed time information to use the time information according to local time of a desired country. Also, the present disclosure is directed to providing a test apparatus for determining whether the validity date of test medium has expired to prevent use of expired test medium and to analyze a test object through available test medium, and a method for controlling the test apparatus. The test apparatus of analyzing a test object included in test medium includes a Real Time Clock (RTC) portion configured to provide current time, a user interface configured to receive a time offset command for changing the current time and displaying the changed current time, a first offset manager configured to reflect the time offset command to the current time provided from the RTC portion to calculate time to be displayed, a second offset manager configured to decide reference time to be used to determine whether validity date of the test medium has expired, based on the current time provided from the RTC portion, a validity date acquirer configured to acquire validity date information of the test medium, and a controller configured to determine whether validity date of the test medium has expired, based on the reference time and the validity date information of the test medium.

## Description

### [Technical Field]

The present disclosure relates to a test apparatus capable of performing in-vitro diagnosis with a small amount of samples.

### [Background Art]

In-vitro diagnosis is technology for determining a patient's health conditions using blood, body fluids, etc. collected from the patient. The in-vitro diagnosis is widely used in prediagnosis for detecting a disease. For in-vitro diagnosis, an immunologic test, a clinical chemical test, etc. are performed on a patient's samples, and the immunologic test and the clinical chemical test play a very important role for diagnosing the patient's status, treating the patient, and determining the prognosis.

A blood analysis apparatus which is a representative example of an in-vitro diagnostic apparatus can perform a blood test quickly and accurately using test medium, such as a disposable cartridge or a disc, and can determine a patient's health conditions with a small amount of blood collected from the patient.

A user uses test medium in order to analyze a test object, such as a patient's blood and body fluids, through an in-vitro diagnostic apparatus. When the user uses the blood analysis apparatus, the user injects blood collected from the patient into the test medium, and then puts the test medium into the blood analysis apparatus to cause the blood analysis apparatus to analyze the blood as a test object. The test medium includes a reagent, etc. for analyzing the test object. For example, the blood analysis apparatus analyzes a reaction of the test object with the reagent included in the test medium to determine whether the patient has specific antibodies or antigens or whether the patient was infected with a specific disease.

In general, test medium has validity date, because of a reagent, etc. included therein. If a test object is analyzed through expired test medium, the results of the analysis may have errors. Accordingly, a user should be careful not to use expired test medium.

Time information displayed on an in-vitro diagnostic apparatus is visually recognized by a user, and the time information needs to be displayed according to local time of a country where the in-vitro diagnostic apparatus is used.

Accordingly, an in-vitro diagnostic apparatus capable of detecting expired test medium in order to analyze a test object through available test medium, while independently managing time information to be displayed is needed.

### [Disclosure]

### [Technical Problem]

The present disclosure is directed to providing an in-vitro diagnostic apparatus for enabling a user to change time information used in the system, and displaying the changed time information to use the time information according to local time of a desired country.

Also, the present disclosure is directed to providing a test apparatus for determining whether the validity date of test medium has expired to prevent use of expired test medium and to analyze a test object through available test medium, and a method for controlling the test apparatus.

### [Technical Solution]

In accordance with one aspect of the present disclosure, a test apparatus of analyzing a test object included in test medium includes: a Real Time Clock (RTC) portion configured to provide current time; a user interface configured to receive a time offset command for changing the current time and displaying the changed current time; a first offset manager configured to reflect the time offset command to the current time provided from the RTC portion to calculate time to be displayed; a second offset manager configured to decide reference time to be used to determine whether validity date of the test medium has expired, based on the current time provided from the RTC portion; a validity date acquirer configured to acquire validity date information of the test medium; and a controller configured to determine whether validity date of the test medium has expired, based on the reference time and the validity date information of the test medium.

The first offset manager may include first time information set to arbitrarily change the current time provided from the RTC portion, based on the time offset command.

The second offset manager may include second time information set to prevent the current time provided from the RTC portion from being arbitrarily changed.

The first offset manager may add a time difference based on the time offset command to a pre-set initial offset to change the current time.

The test apparatus may further include: a display configured to display the time calculated by reflecting the time offset command, the display may display information informing that the validity date of the test medium has expired, when the validity date of the test medium has expired.

The validity date acquirer may acquire the validity date information of the test medium by recognizing at least one of a barcode, a Quick Response (QR) code, text data, a data matrix, a recognition pattern, Near Field Communication (NFC), and Radio Frequency Identification (RFID) that are installed in the test medium and include information about the test medium.

The validity date acquirer may include: a sensor configured to recognize at least one of a barcode, a Quick Response (QR) code, text data, a data matrix, a recognition pattern, Near Field Communication (NFC), and Radio Frequency Identification (RFID).

The controller may compare the validity date information of the test medium to the reference time to determine whether the validity date of the test medium has expired.

The test apparatus may further include: an analyzer may analyze the test object included in the test medium if the validity date of the test medium has not expired.

The analyzer may not analyze the test object included in the test medium if the validity date of the test medium has expired.

The test apparatus may further include: a communication device may receive the current time information from an external server.

The test medium may include at least one of a disc and a cartridge.

In accordance with another aspect of the present disclosure, a method of controlling a test apparatus includes: receiving current time; receiving a time offset command for changing the current time and displaying the changed current time; reflecting the time offset command to the current time to calculate time to be displayed; deciding reference time to be used to determine whether validity date of the test medium has expired, based on the current time; acquiring validity date information of the test medium; determining whether the validity date of the test medium has expired, based on the reference time and the validity date information of the test medium.

The reflecting of the time offset may command to the current time to calculate the time to be displayed comprises adding a time difference based on the time offset command to a pre-set initial offset to change the current time.

The method of controlling a test apparatus may further include: displaying the time calculated by reflecting the time offset command.

### [Advantageous Effects]

According to an embodiment of the present disclosure, it is possible to strictly determine expiration of the validity date of test medium corresponding to a consumable in in-vitro diagnosis.

Also, since a user can change time to be displayed on an in-vitro diagnostic apparatus, the user will not experience any inconvenience in using the in-vitro diagnostic apparatus in different countries.

### [Description of Drawings]

FIG. 1 is a perspective view showing an outer appearance of a test apparatus according to an embodiment of the present disclosure, and FIG. 2 shows an outer appearance of test medium according to an embodiment of the present disclosure which is inserted into the test apparatus of FIG. 1.
FIG. 3 is a control block diagram showing a configuration of the test apparatus according to the embodiment of the present disclosure.
FIG. 4 is a conceptual view for describing operation for changing current time displayed on the test apparatus, according to an embodiment.
FIG. 5 is a conceptual view for describing operation for setting second time information stored in a second offset manager, according to an embodiment.
FIG. 6 is a view for describing test medium according to an embodiment.
FIG. 7 is a view for describing a notification message displayed on a display, according to an embodiment.
FIG. 8 is a view for describing operation in which the test apparatus according to an embodiment determines the validity date of test medium.
FIG. 9 is a perspective view showing an outer appearance of a test apparatus according to another embodiment of the present disclosure.
FIG. 10 is a flowchart illustrating a method of controlling a test apparatus to change and display current time, according to an embodiment, and FIG. 11 is a flowchart illustrating a method of controlling a test apparatus to determine whether the validity date of test medium has expired, according to an embodiment.

### [Modes of the Invention]

Advantages and features of the present disclosure and a method and apparatus of achieving the advantages and features will be apparent by referring to embodiments described below in connection with the accompanying drawings. However, the present disclosure is not restricted by these embodiments but can be implemented in many different forms. The present embodiments are provided to complete the disclosure of the present invention and to allow those having ordinary skill in the art to understand the scope of the present disclosure. The present disclosure is defined by the category of the claims.

Terms used in this specification will be briefly described, and the present disclosure will be described in detail.

Although general terms being widely used at the present disclosure were selected as terminology used in the present disclosure while considering the functions of the present disclosure, they may vary according to intentions of one of ordinary skill in the art, judicial precedents, the advent of new technologies, and the like. Terms arbitrarily selected by the applicant of the present disclosure may also be used in a specific case. In this case, their meanings need to be given in the detailed description of the present disclosure. Hence, the terms must be defined based on the meanings of the terms and the contents of the entire specification, not by simply stating the terms themselves.

It will be understood that when the terms "includes," "comprises," "including," and/or "comprising," when used in this specification, specify the presence of stated elements and/or components, but do not preclude the presence or addition of one or more elements and/or components thereof. As used herein, the terms "part", "module", or "unit" refers to a unit that can perform at least one function or operation, and may be implemented as a software or hardware component such as a Field Programmable Gate Array (FPGA) or an Application Specific Integrated Circuit (ASIC). However, the term "part", "module" or "unit" is not limited to software or hardware. The "part", "module", or "unit" may be configured in an addressable storage medium, or may be configured to run on at least one processor. Therefore, as an example, the "part", "module", or "unit" includes: components such as software components, object-oriented software components, class components, and task components; processors, functions, attributes, procedures, sub-routines, segments of program codes, drivers, firmware, microcodes, circuits, data, databases, data structures, tables, arrays, and variables. Functions provided in the components and the "part", "module", or "unit" may be integrated into the smaller number of components and the "part", "module", or "unit", or may be sub-divided into additional components and an additional "part", "module", or "unit".

Hereinafter, embodiments of the present disclosure will be described in detail with reference to the accompanying drawings so that the present disclosure may be readily implemented by those skilled in the art. However, the present disclosure can be implemented in different forms, and is not limited to the embodiments which will be described below. In the drawings, parts irrelevant to the description are omitted for the simplicity of explanation.

In this specification, the term "user" may be a medical specialist including a paramedic, a doctor, a nurse, a medical technologist, and a radiological technologist, and may also be an engineer who repairs medical equipment. However, the user is not limited to the above-mentioned persons.

Also, although a test apparatus which will be described below is not limited to an in-vitro diagnostic apparatus, a case in which the test apparatus is an in-vitro diagnostic apparatus will be described as an embodiment of the present disclosure.

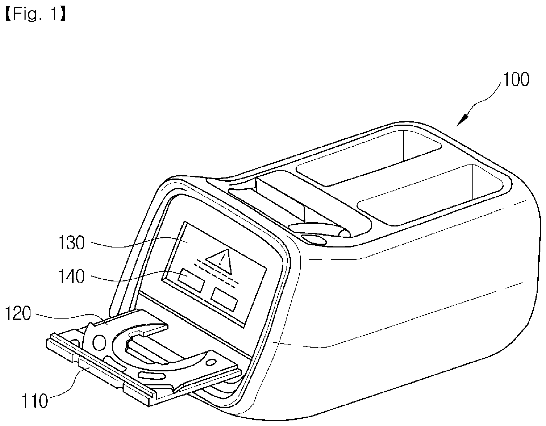

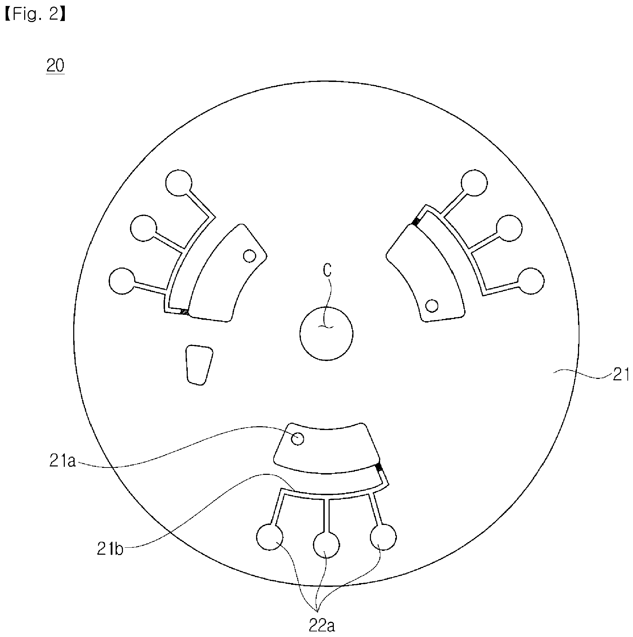

FIG. 1 is a perspective view showing an outer appearance of a test apparatus according to an embodiment of the present disclosure, and FIG. 2 shows an outer appearance of test medium according to an embodiment of the present disclosure which is inserted into the test apparatus of FIG. 1.

A test apparatus 100, for example, an in-vitro diagnostic apparatus 100 may be a blood tester. Hereinafter, a case in which the test apparatus 100 according to an embodiment of the present disclosure is a blood tester will be described as an example. Meanwhile, the test apparatus 100 to analyze a test object contained in test medium is classified into a cartridge type in which a sample or a reagent is moved by a capillary force, a disc type in which a sample or a reagent is moved by a centrifugal force, and a cuvette type in which measurement is performed without movement of a sample or a reagent. The structure or configuration of a test apparatus depends on its type, and the embodiment shown in FIG. 1 shows the test apparatus 100 into which disc type test medium 20 is inserted.

The test apparatus 100 may be constructed as shown in FIG. 1.

The test apparatus 100 may receive test medium 20 including blood collected from a patient and placed on a test medium inserting surface 120 of a loader 110, analyze the blood included in the test medium 20, and output the results of the analysis through a display 130.

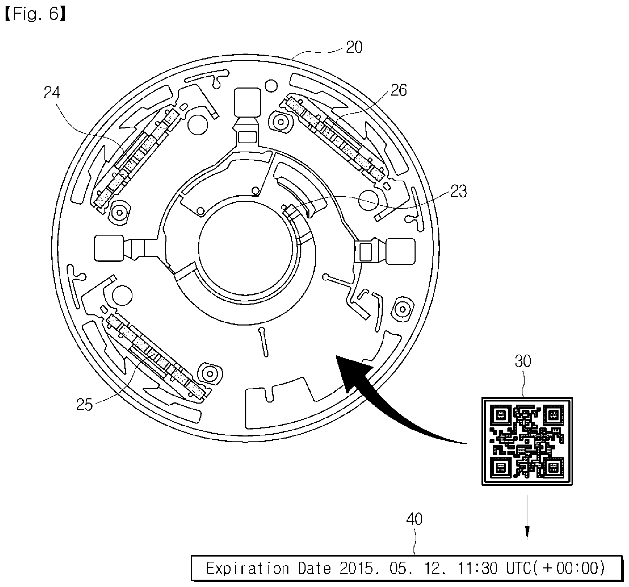

The test medium 20 may be configured to include blood as an object that is to be tested. The test medium 20 may be a disc type, a cartridge type, etc. The test medium 20 according to an embodiment of the present disclosure will be described in detail with reference to FIG. 6, below.

Also, the test apparatus 100 may start analyzing the blood as a test object according to a predetermined trigger signal informing a test start. For example, the display 130 may be implemented as a touch screen in which a display panel is combined with a touch panel. In this case, a user interface screen which is a menu screen for performing a blood test may be displayed through the display 130. A user may touch a predetermined button included in the menu screen to start a blood test.

Also, the test apparatus 100 may have a portable size, as shown in FIG. 1, and the test apparatus 100 may be installed in transportation means, such as an ambulance or a rescue helicopter, to transport an emergency patient.

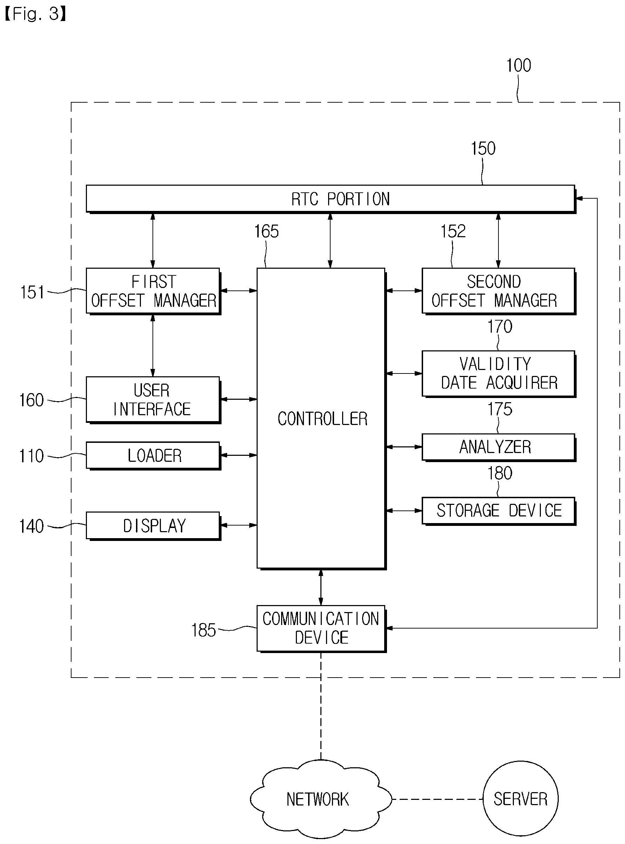

The test apparatus 100 may determine validity date of the test medium 20 before starting a blood test. A method in which the test apparatus 100 acquires information of validity date of the test medium 20 and determines whether the validity date of the test medium 20 has expired will be described with reference to FIGS. 3 and 6, later.

If expired test medium is used, a reagent and a test object included in the test medium may react abnormally so as to generate errors in the results of analysis on the test object. In this case, patients or carers may make an objection to the results of the analysis. Accordingly, a user should be careful not to use expired test medium.

However, there may be a case in which a user uses expired test medium by intention due to economic reasons such as cost reduction. Also, if a user can arbitrarily set time information of the test apparatus 100, there is no method for preventing such misuse of the system of the test apparatus 100.

Also, if time information of the test apparatus 100 is set based on local time of a region where the test apparatus 100 is located, a plurality of test apparatus 100 located in different regions may make different determinations on whether the validity date of the same test medium 20 has expired. For example, a case in which a test apparatus 100 located in Korea is set to local time of +9:00 from Universal Time Coordinated (UTC), and a test apparatus 100 located in England is set to local time of +00:00 from UTC is assumed. In this case, the test apparatus 100 located in Korea may determine that the validity date of test medium 20 has expired, and the test apparatus 100 located in England may determine that the validity date of the same test medium 20 has not yet expired.

A case in which the expired test medium 20 is determined as available test medium according to a region where the test apparatus 100 is located may be more problematic than the inverse case.

Also, there may be a case in which a user uses expired test medium 20 unintentionally due to a mechanical defect. For example, in the case in which an error is generated in time control system of the test apparatus 100, a user may use expired test medium 20.

However, a user may need to set time information of the test apparatus 100 to arbitrary time, as necessary or on purposes. For example, when time information of the test apparatus 100 has been set to local time of a region where it was released, the user may need to set the time information of the test apparatus 100 to local time of a region where he or she is located. Also, the user may need to set time information of the test apparatus 100 to arbitrary time, in order to record predetermined time information in the result of analysis or on an analysis report of the test apparatus 100.

Accordingly, the present disclosure provides the test apparatus 100 that can prevent a case in which a user misuses expired test medium 20, a case in which the test apparatus 100 determines the validity date of the test medium 20 wrongly according to local time of a region where it is located, and a case in which a user uses expired test medium 20 due to a mechanical defect. In addition, the present disclosure provides the test apparatus 100 to allow a user to set time information to arbitrary time as necessary.

Referring to FIG. 2, the disc type test medium 20 may be configured with a rotatable platform 21, and a plurality of structures formed on the platform 21. The structures may include a plurality of chambers to accommodate a test object or a reagent, and a plurality of channels connecting the chambers to each other. The structures may be formed in the inside of the test medium 20 such that a user can see the structures from above since the test medium 20 is made of a transparent material.

The platform 21 may be made of a material which can be easily molded and whose surface is biologically inert. The material may include a plastic material (for example, acrylic (PMMA), polymethylalkylsiloxane (PDMS), polycarbonate (PC), polypropylene (PP), polyvinyl alcohol (PVA), and polyethylene (PE)), glass, mica, silica, a silicon wafer, and the like.

However, the platform 21 may be made of any other material as long as it has chemical and biological stability and machinability. If test results need to be optically analyzed in the test medium 20, the platform 21 may further have optical transparency.

In the platform 21, an inlet hole 21a into which a sample is injected, a chamber 22a in which a reagent is contained, and a channel 21b connecting the inlet hole 21a to the chamber 22a may be formed.

The test apparatus 100 may rotate the test medium 20. A turn table for transferring a rotational force provided by the test apparatus 100 may be inserted in a center hole C formed in the center of the test medium 20. When the test medium 20 rotates, a test object injected through the inlet hole 21a may move to the chamber 22a by a centrifugal force. If the test object is blood, the blood may be centrifugally separated when the test medium 20 rotates. Accordingly, the platform 21 may further include a plurality of structures for centrifugal separation of blood.

In the disc type test medium 20, the platform 21 may be configured with a plurality of layered plates. If the platform 21 is configured with two plates of an upper plate and a lower plate, a plurality of structures corresponding to chambers, channels, etc. may be engraved in the surfaces of the upper and lower plates contacting each other, and then the upper plate may be joined with the lower plate to thereby form spaces and passages to contain and move fluid in the inside of the platform 21. The upper and lower plates may be joined by various methods, such as adhesion using an adhesive or a double-sided tape, ultrasonic-waves welding, and laser welding.

Meanwhile, the test medium 20 can induce quantitative analysis with a small amount of a test object. Also, a test object or a reagent moving along a channel in the test medium 20 may be fluid. Accordingly, the test medium 20 is also called a microfluidic device.

Since a plurality of chambers 22a are provided to accommodate different reagents, various inspection tests can be conducted simultaneously.

For example, the chambers 22a may contain a GGT test reagent, a CREA test reagent, a TRIG test reagent, a CHOL test reagent, and an ALT test reagent, respectively, to simultaneously perform a CCT test, a CREA test, a TRIG test, a CHOL test, and an ALT test.

FIG. 3 is a control block diagram showing a configuration of the test apparatus according to the embodiment of the present disclosure.

Referring to FIG. 3, the test apparatus 100 may include the loader 110, a Real Time Clock (RTC) portion, a first offset manager 151, a second offset manager 152, a user interface 165, a controller 170, a validity date acquirer 175, an analyzer 180, a storage device 185, and a communication device 185.

The loader 110 may receive the test medium 20. The test medium 20 may be medium including a test object, such as blood and body fluids. More specifically, the loader 110 may include a medium inserting portion to receive the test medium 20, and the shape of the loader 110 may depend on the test medium 20.

In FIG. 1, a case in which the test medium 20 is a disc type is shown as an example. In this case, the loader 110 may include, as the medium inserting portion, a disc tray (not shown) into which a disc can be inserted.

That is, the loader 110 may drive a disc tray so that the disc tray is drawn to enable a user to mount a disc on the disc tray. According to another embodiment, the test medium 20 may be a cartridge type. In this case, the loader 110 may have a predetermined shape into which a cartridge can be inserted.

That is, the loader 110 may have various shapes depending on the shapes of the test medium 20.

The RTC portion 150, which is a real time clock, may be installed in the inside of the test apparatus 100 to provide information of current time. That is, the RTC portion 150 may receive standard time when the test apparatus 100 is manufactured or when control system of the test apparatus 100 is set initially, store the standard time in an internal register, and output the standard time as current time.

The current time provided by the RTC portion 150, which is time displayed on the test apparatus 100, may be recognized by a user, and provide time information that is used to determine whether the validity date of the test medium 20 has expired. Since time displayed on the test apparatus 100 needs to change according to a country where the test apparatus 100 is used or according to local time at which the test apparatus 100 is used, a user had to change current time information through the RTC portion 150 to determine the validity date of the test medium 20 based on the changed time information.

In this case, there was a problem that the user changes the current time information to manipulate reference time to be used to determine the validity date.

In order to avoid the problem, a technique of receiving reference time information to be used to determine the validity date from an external server through a network to determine the validity date of test medium based on the received time information has been developed. However, there is a limitation that the test apparatus should connect to a network in order to receive time information from an external server.

In order to remove the limitation, another technique of using two RTC portions to enable a user to change time information to be displayed on a test apparatus and checked by the user through one RTC portion, and to set reference time to be used to determine the validity date of test medium through the other RTC portion has been developed. However, use of two RTC portions results in an increase of manufacturing cost of the test apparatus.

Accordingly, the test apparatus 100 according to an embodiment of the present disclosure is characterized that a single RTC portion 150 is used to manage reference time for determining the validity date of the test medium 20 separately from time displayed on the test apparatus 100 to be checked by a user.

The RTC portion 150 may include a RTC circuit, and the RTC circuit may be a circuit that is widely used to provide time information to a PC, a server, embedded system, etc.

The RTC portion 150 may provide initially set current time information to the first offset manager 151 and the second offset manager 152, and may receive current time information from an external server connected to an external network through communication with the communication device 185. The current time information set in the RTC portion 150 cannot be changed by a general user, and can be set and changed by a manufacturer or an engineer when the test apparatus 100 is manufactured or when the RTC portion 150 is installed.

The user interface 160 may receive a time offset command for changing time information that is displayed on the test apparatus 100 to be checked by a user, from the user. That is, since time displayed on the test apparatus 100 needs to be changed based on current time information provided from the RTC portion 150, according to a country where the test apparatus 100 is used or according to local time at which the test apparatus 100 is used, the user may input a time offset command for changing time information that is to be displayed on the test apparatus 100, through the user interface 160. The time offset command input by the user may be transferred to the controller 165 and the first offset manager 151, and the first offset manager 151 may calculate time that is to be displayed on the test apparatus 100, based on the time offset command.

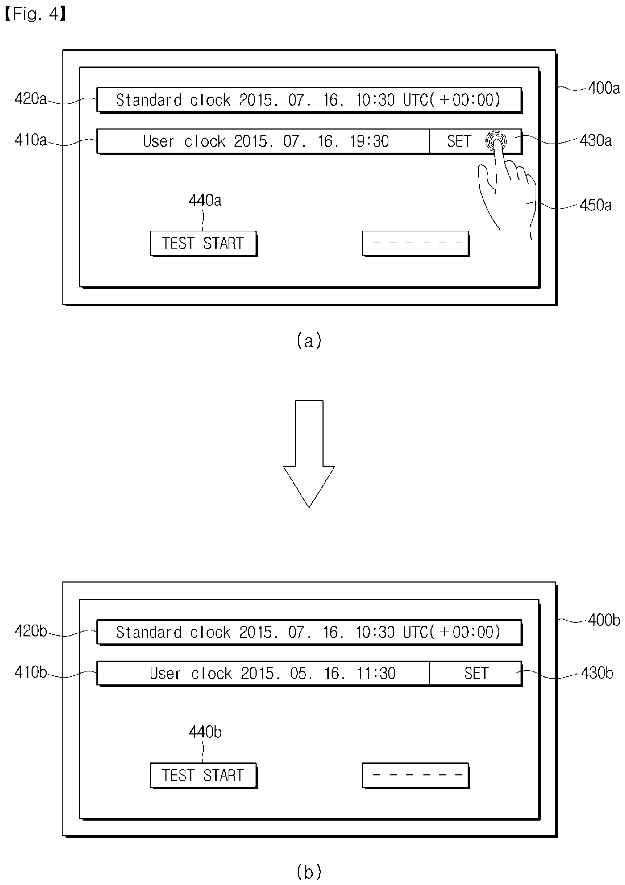

A user interface screen to enable the user to input a time offset command will be described in detail with reference to FIG. 4, later.

The user interface 160 may include a device for receiving a predetermined input from the outside. For example, the user interface 160 may be a mouse, a keyboard, or an input device including hard keys for inputting predetermined data.

Also, the user interface 160 may be a touch pad. More specifically, the user interface 160 may be a touch pad (not shown) combined with a display panel (not shown) included in the display 140. Also, the display 140 may display a user interface screen on the display panel. If a user touches a predetermined point on the user interface screen to input a predetermined command, the touch panel may recognize the user's touch on the predetermined point to recognize the predetermined command input by the user.

More specifically, if the user interface 160 is a touch pad, a user may touch a predetermined point on a user interface screen. Then, the user interface 160 may sense the point touched by the user, and transmit the result of the sensing to the controller 165. Then, the controller 165 may recognize the user's request or command corresponding to the result of the sensing, and perform the recognized request or command.

The user may manipulate at least one of a mouse, a keyboard, a touch pad, or another input device included in the user interface 160 to input a time offset command for changing time information to be displayed on the test apparatus 100.

The first offset manager 151 may reflect the time offset command received from the user to the current time information provided from the RTC portion 150 to calculate time to be displayed.

The first offset manager 151 may store first time information set to arbitrarily change the current time provided from the RTC portion 150 based on the time offset command received from the user.

As described above, the user may need to set time information of the test apparatus 100 to arbitrary time information as necessary or on purposes. For example, when the test apparatus 100 used in a certain country is used in another country, or when the test apparatus 100 manufactured in a certain country is used in another country, a user may need to set time information of the test apparatus 100 according to local time. Accordingly, the test apparatus 100 according to an embodiment of the present disclosure may include the first offset manager 151 to change current time based on current time information received from the RTC portion 150 and an offset command received through the user interface 160.

The first offset manager 151 may store the first time information which is information of current time received from the RTC portion 150, wherein the first time information can be changed according to a time offset command input by a user.

The first offset manager 151 may add a time difference based on the time offset command received from the user to a pre-set initial offset to change the current time, thereby calculating time to be displayed.

For example, the first offset manager 151 may initially set a time offset to "0", and store the time offset "0" therein. Thereafter, if a user inputs a time offset command through the user interface 160, the first offset manager 151 may add a difference from a time offset value input by the user to the set time offset to change time information provided from the RTC portion 150. That is, if current time provided from the RTC portion 150 is 2015.08.14, the current time of 2015.08.14 may correspond to first time information, and if a time offset command input by the user is 2015.08.17, the first offset manager 151 may add "3" corresponding to the difference to the initially set time offset "0", and change time to be displayed to 2015.08.17.

The initial offset setting value and the offset value of the changed time may be stored in the first offset manager 151 or in the storage device 180.

The time information changed by the user may be displayed on the display 140 so as to be checked by the user, which will be described later.

The second offset manager 152 may store current time information provided from the RTC portion 150 as second time information, and decide reference time to be used to determine whether the validity date of the test medium 20 has expired, based on the second time information. The second time information, which is time information provided from the RTC portion 150, cannot be changed arbitrarily by a user.

Unlike the first offset manager 151 and the first time information that is provided from the RTC portion 150 and stored in the first offset manager 151 and that can be changed when a user inputs an offset command, the second time information stored in the second offset manager 152 cannot be set or changed arbitrarily by a general user, since the second time information corresponds to the same time information as current time provided by the RTC portion 150.

The second time information stored in the second offset manager 152 may correspond to reference time to be used to determine whether the validity date of the test medium 20 has expired, wherein the reference time may be the same time information as that applied to set the validity date of the test medium 20. For example, the reference time to be used to determine whether the validity date of the test medium 20 has expired may be time information set by a manufacturer of the test medium 20. The second time information corresponding to the reference time cannot be changed arbitrarily by a general user or consumer. However, a manager, such as a manufacturer of the test apparatus 100 or an engineer, who has authority of access to the control system of the test apparatus 100, can manipulate the RTC portion 150 or the second offset manager 152 to change the second time information.

The second time information stored in the second offset manager 152 cannot be set or changed to arbitrary time that is not current time provided by the RTC portion 150. As described above, if the test apparatus 100 determines whether the validity date of the test medium 20 has expired based on time information that can be set to arbitrary time, a user's intentional misuse cannot be prevented. Accordingly, unlike the first time information and the first offset manager 151 for changing time information displayed on the test apparatus 100, the second time information stored in the second offset manager 152 may be provided neither a user interface screen nor a physical button for allowing a general user to change the information.

That is, a general user may input a time offset command through the user interface 160, and the first offset manager 151 may calculate time to be changed and displayed, based on the first time information included therein, according to the time offset command. However, the general user cannot input a control command for changing the second time information stored in the second offset manager 152 to determine the validity date of the test medium 20, and the second time information stored in the second offset manager 152 may be changed restrictively by a manufacturer or an engineer.

As a result, current time information provided from the RTC portion 150 may be stored as first time information that can be arbitrarily changed, in the first offset manager 151, and the first time information may be changed by a user to change time to be displayed on the test apparatus 100. Simultaneously, the current time information may be stored as second time information that cannot be arbitrarily changed, in the second offset manager 152, and the second time information may be decided as reference time to be used to determine whether the validity date of the test medium 20 has expired.

The second time information stored in the second offset manager 152 may be current time information received from the RTC portion 150, and may be set to reference time based on UTC. Also, validity date recorded on the test apparatus 100 may be set based on UTC. Accordingly, the controller 165 may determine whether the validity date of the test apparatus 100 has expired, in consideration of a difference between local time information of the second time information and local time information used to set the validity date of the test apparatus 100, based on UTC.

The validity date acquirer 170 may be installed in the test medium 20 to recognize a barcode, a Quick Response (QR) code, text data, a data matrix, or a recognition pattern including information about the test medium 20, or the validity date acquirer 170 may perform short-range communication, such as Near Field Communication (NFC) and Radio Frequency Identification (RFID), with the test medium 20 to acquire validity date information of the test medium 20. That is, the validity date acquirer 170 may include a sensor to recognize at least one of a barcode, a QR code, text data, a data matrix, a recognition pattern, NFC, and RFID.

Also, the validity date acquirer 170 may acquire information of at least one of a test object and the test medium 20 by the same method. For example, the validity date acquirer 170 may recognize a barcode, a QR code, etc. attached on the surface of the test medium 20 to acquire physical conditions about an examinee of a test object, such as date of birth, race, height, and weight, and to acquire a unique identification number, manufacturing date, etc. of the test medium 20.

The validity date information of the test medium 20, acquired by the validity date acquirer 170 may be transferred to the controller 165 or stored in the storage device 180.

The controller 165 may control the display 140 to display the time calculated by the first offset manager 151.

Also, the controller 165 may determine whether the validity date of the test medium 20 has expired, based on the reference time decided by the second offset manager 152 and the validity date information of the test medium 20 acquired by the validity date acquirer 170.

More specifically, the controller 165 may compare the validity date information of the test medium 20 acquired by the validity date acquirer 170 to the reference time decided by the second offset manager 152. If the controller 165 determines that time included in the validity date information of the test medium 20 is earlier than the reference time decided by the second offset manager 152, the controller 165 may determine that the validity date of the test medium 20 has expired, and otherwise, the controller 165 may determine that the validity date of the test medium 20 has not expired.

Also, the controller 165 may control analysis of the test object based on the determination on whether the validity date of the test medium 20 has expired.

That is, the controller 165 may drive the validity date acquirer 170 before starting analyzing the test object, to acquire validity date of the test medium 20 received through the loader 110, and if the controller 165 determines that the validity date of the test medium 20 has not expired, the controller 165 may transfer a predetermined trigger signal informing a test start to control the analyzer 175 to start analyzing the test object.

If the validity date of the test medium 20 has not expired, the analyzer 175 may analyze the test object included in the test medium 20 under the control of the controller 165. The analyzer 175 may analyze the test object such as blood to create the result of analysis for determining whether the corresponding patient was infected with a predetermined disease.

That is, if it is determined that the validity date of the test medium 20 has expired, based on the reference time decided by the second offset manager 152, the analyzer 175 may not analyze the test object included in the test medium 20.

Also, when the analyzer 175 analyzes the test object according to the control of the controller 165, time information used in the analysis and time information recorded on the result of the analysis may be time information calculated by the first offset manager 151.

When current time provided from the RTC portion 150 is changed, the display 140 may display the changed time. More specifically, the display 140 may display time calculated by reflecting a time offset command received through the user interface 160 to current time provided from the RTC portion 150, so that a user can use the test apparatus 100 according to time information of a desired country based on the displayed time.

Also, if the validity date of the test medium 20 has expired, the display 140 may display a notification message informing the expiration of the validity date. More specifically, if the validity date of the test medium 20 has expired, the controller 165 may create a notification message informing the expiration of the validity date, and the display 140 may display the notification message. The display 140 may display the notification message as a visual signal, or output the notification message as an auditory signal.

The display 140 may display an interface screen for time information changed by the user or for a notification message informing the expiration of the validity date, on the display panel. The time information and the notification message displayed on the display 140 will be described in detail with reference to FIGS. 4 to 7, below.

The communication device 185 may be connected to a network in a wired or wireless fashion to communicate with an external server, external medical equipment, or an external device.

For example, the communication device 185 may transmit the result of analysis on a test object to an external server through a network, or receive information about at least one of the test medium 20 and a test object from an external server through the network.

Also, the communication unit 185 may receive current time information from an external server, and transfer the current time information to the RTC portion 150. The controller 165 may set at least one of the first time information stored in the first offset manager 151 or the second time information stored in the second offset manager 152, based on the current time information received from the communication device 185. That is, when an error is generated in at least one of the first offset manager 151 or the second offset manager 152, or when at least one of the first time information or the second time information becomes different from current time information provided from the RTC portion 150 due to the physical characteristics of at least one of the first offset manager 151 or the second offset manager 152, the first time information or the second time information may need to be updated.

Accordingly, the test apparatus 100 according to an embodiment may receive current time information from an external server, etc. through the communication device 185, and update at least one of the first time information or the second time information based on the received current time information through the controller 165. At least one of the first time information or the second time information may be updated at regular time intervals, when the communication device 185 is connected to the network, or according to a user's command.

The storage device 180 may store various data, programs, etc. required for analyzing the test medium 20. Also, the storage device 180 may be at least one type of storage medium among a flash memory type, a hard disk type, a multimedia card micro type, card type memory (for example, Secure Digital (SD) or eXtreme Digital (XD) memory), Random Access Memory (RAM), Static Random Access Memory (SRAM), Read-Only Memory (ROM), Electrically Erasable Programmable Read-Only Memory (EEPROM), Programmable Read-Only Memory (PROM), magnetic memory, a magnetic disk, and an optical disk. More specifically, the storage device 180 may store the progress of a blood test, test results, etc.

Also, the storage device 180 may store the first time information stored in the first offset manager 151, the second time information stored in the second offset manager 152, and information about the validity date of the test medium 20 acquired by the validity date acquirer 170.

FIG. 4 is a conceptual view for describing operation for changing current time displayed on the test apparatus, according to an embodiment.

User interface screens 400a and 400b of FIG. 4 may be displayed on the display 140 shown in FIG. 3.

The user interface screens 400a and 400b of FIG. 4 may include first time information 410a and 410b stored in the first offset manager 151, second time information 420a and 420b stored in the second offset manager 152, icons 430a and 430b for setting the first time information 410a and 410b, and trigger buttons 440a and 440b for generating a predetermined trigger signal informing a start of a test for analyzing the test medium 20.

The first time information 410a and 410b may be set to arbitrary time by a user. For example, as shown in (a) of FIG. 4, the first time information 410a may be set to UTC (+9:00) which is local time of Korea, when the user is located in Korea.

The second time information 420a and 420b may be based on current time information provided from the RTC portion 150. For example, the second time information 420a and 420b may be set to 2015.07.16 10:30 UTC (+00:00) which is UTC-based reference time. More specifically, the second time information 420a and 420b may be set to UTC (+00:00) of England. Since the reference time includes local time information, the result of determination on whether the validity date of the test medium 20 has expired may be not influenced by UTC of regions.

Also, the controller 165 may determine whether the validity date of the test medium 20 has expired, based on the second time information 420a and 420b.

The analyzer 175 may analyze a test object based on the first time information 410a and 410bn according to the control of the controller 165. For example, time information recorded on the result of analysis of the test apparatus 100 may be the first time information 410a and 410b.

A user input 450a shown in (a) of FIG. 4 may be an input for changing current time provided from the RTC portion 150 based on the first time information 410a. More specifically, the user interface 160 of FIG. 3 may receive an input for changing the first time information 410a, and the controller 165 may change 2015.07.16 19:30 which is the first time information 410a shown in (a) of FIG. 4 to 2015.05.16 11:30 which is the first time information 410b shown in (b) of FIG. 4, according to the received input.

At this time, the user may input a time offset command for changing time, through the user interface 160, or the first offset manager 151 storing the first time information 410a may add a difference from the time offset value received from the user to a time offset stored upon initial setting to calculate time. The first offset manager 151 may transfer information of the calculated time to the controller 165, and the controller 165 may control the display 140 to display the time input by the user.

The trigger buttons 440a and 440b may generate a predetermined trigger signal informing a start of a test for analyzing a test object included in the test medium 20. For example, if the user interface 140 receives a user input of pressing the trigger buttons 440a and 440b, the controller 165 may drive the loader 110 to receive the test medium 20. Also, the validity date acquirer 170 may acquire validity date of the received test medium 20. The controller 165 may control the analyzer 175 according to whether the validity date of the test medium 20 has expired.

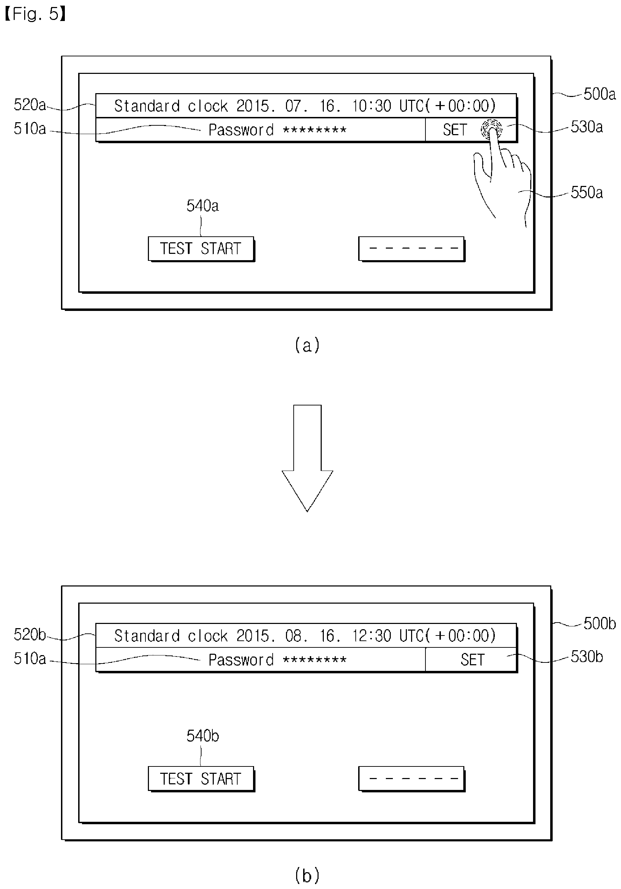

FIG. 5 is a conceptual view for describing operation for setting second time information stored in a second offset manager, according to an embodiment.

As described above, current time information set in the RTC portion 150 cannot be arbitrarily set by a general user, and may be set and changed restrictively by a manufacturer or an engineer when the test apparatus 100 is manufactured or when the RTC portion 150 is installed.

Also, the second time information stored in the second offset manager 152 may be information based on current time provided from the RTC portion 150, and correspond to reference time to be used to determine whether the validity date of the test medium 20 has expired. That is, reference time to be used to determine whether the validity date of the test medium 20 has expired may be time information set by a manufacturer of the test medium 20, and accordingly, the second time information corresponding to the reference time cannot be arbitrarily changed by a general user or consumer. However, a manager, such as a manufacturer of the test apparatus 100 or an engineer for the test apparatus 100, who has authority of access to the control system of the test apparatus 100, can manipulate the RTC portion 150 or the second offset manager 152 to change the second time information.

User interface screens 500a and 500b of FIG. 5 may be displayed by the display 140 of FIG. 3.

The user interface screens 500a and 500b of FIG. 5 may include second time information 520a and 520b stored in the second offset manager 152, icons 510a 510b to enable a manufacturer or an engineer to input a password in order to change the second time information 520a and 520b, and icons 530a and 530b to set the second time information 520a and 520b.

A general user cannot set the second time information 520a, and only a manager, such as a manager or an engineer, can manipulate the icon 510a for setting the second time information 520a after inputting a password for setting the second time information 520a. The manager, such as a manufacturer or an engineer, may change current time information provided from the RTC portion 150 by changing the design of the RTC portion 150 or by resetting the current time information. Also, the manager can change the second time information 520a stored in the second offset manager 152, as described above. As shown in (a) of FIG. 5, the manager may input a manipulation command through the user interface 160 in order to change the second time information 520a.

As shown in (a) of FIG. 5, the user interface 160 may receive an input for changing the second time information 520a, and the controller 165 may change 2015.07.16 10:30 which is the second time information 520a shown in (a) of FIG. 5 to 2015.08:16 12:30 which is the second time information 520b shown in (b) of FIG. 5, according to the received input.

That is, the second time information 520b changed by the manufacturer or the engineer may be stored in the second offset manager 152, and whether the validity date of the test medium 20 has expired may be determined based on the second time information 520b.

FIG. 6 is a view for describing test medium according to an embodiment.

If the test medium 20 is a disc for blood test, the disc for blood test may be constructed as shown in FIG. 6.

Referring to FIG. 6, blood collected from a patient may be injected into the inside of the test medium 20 through an injection hole 23 of the disc for blood test which is the test medium 20. The injected blood may spread to one or more strips 24, 25, and 26 included in the disc for blood test.

Also, the disc for blood test which is the test medium 20 may include identification information 30 which is information for identifying the test medium 20. The identification information 30 may be implemented as a QR code as shown in FIG. 6, and attached on a front surface of the disc for blood test.

The analyzer 175 may analyze a test object included in the test medium 20. For example, the analyzer 175 may perform detailed analysis on the blood existing in the strips 24, 25, and 26 included in the disc for blood test which is the test medium 20.

The analyzer 175 may perform a predetermined test on the test object. The analyzer 175 may perform a predetermined test for determining whether the patient was infected with a predetermined disease, based on the identification information 30. Also, the analyzer 175 may perform a predetermined test for detecting a predetermined disease, based on a user's settings or initial settings of the test apparatus 100.

For example, the analyzer 175 may perform a Troponin I (Tnl) test on the blood as a test object. Herein, the Tnl test is a cardiac marker test that is used to diagnose an Acute Coronary Syndrome (ACS) including Acute Myocardiac Infarction (AMI). An emergency patient suspected of myocardial infarction needs to take a Tnl test as a cardiac marker test. Tni is an indicator of myocardial injury, and appears in the blood when myocardial tissue is damaged.

The identification information 30 of the test medium 20 may include information about at least one of the test object and the test medium 20. Also, the identification information 30 of the test medium 20 may include validity date information 40 of the test medium 20. For example, the validity date of the disc for blood test as shown in FIG. 6 may be 2015.05.12 11:30 UTC (+00:00).

Also, the validity date acquirer 170 may recognize the identification information 30 which is a QR code to acquire the validity date of the test medium 20. Also, the validity date acquirer 170 may recognize the identification information 30 which is a QR code to acquire physical conditions about an examinee of a test object, such as date of birth, race, height, and weight, and to acquire a unique identification number, manufacturing date, etc. of the test medium 20.

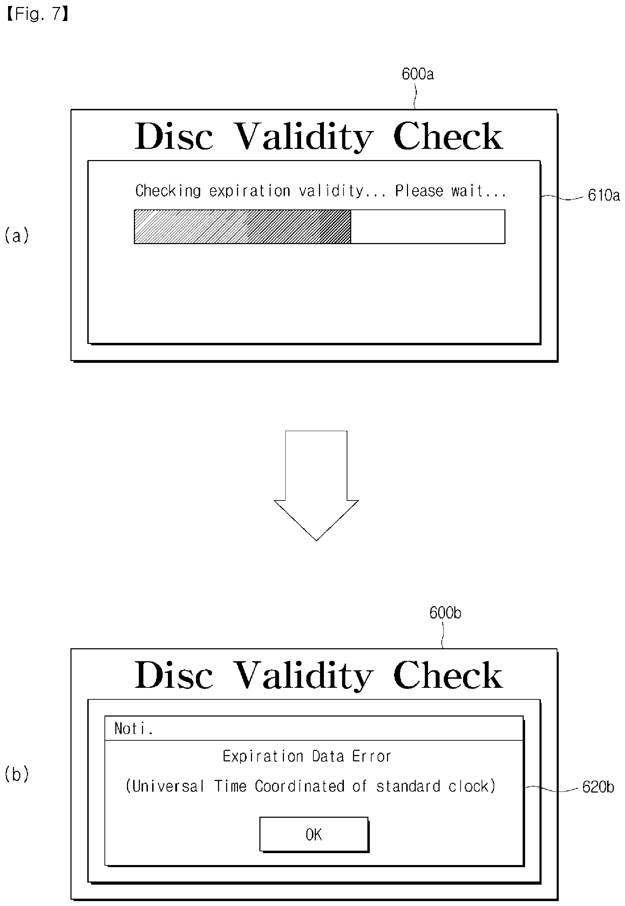

FIG. 7 is a view for describing a notification message displayed on a display, according to an embodiment.

More specifically, (a) of FIG. 7 shows a user interface screen 600a that displays a notification message 610a informing that it is being determined whether the validity date of the test medium 20 has expired, and (b) of FIG. 7 shows a user interface screen 600b that displays a notification message 620b informing that the validity date of the test medium 20 has expired.

More specifically, the user interface screen 600a including the notification message 610a informing that the validity date of the test medium 20 is being determined may be displayed on the display 140, until it is determined whether the validity date of the test medium 20 has expired based on second time information from when a trigger signal informing a test start is input.

If the controller 165 determines that the validity date of the test medium 20 has expired, the controller 165 may generate a notification message representing expiration of the validity date, and the display 140 may output the notification message.

The notification message may include a message representing expiration of the validity date, or a user display screen 620b such as a pop-up window. Also, the notification message may include a visual signal such as LED, or an auditory signal such as warning sound.

For example, there is a case in which the validity date (2015.05.12 11:30 UTC (+00:00)) 40 of the disc for blood test of FIG. 6 has expired based on the second time information (2015.07.16 10:30 UTC (+00:00)) 420a and 420b of FIG. 4. In this case, the controller 165 may generate a notification message representing expiration of the validity date, regardless of the first time information 410a and 410b.

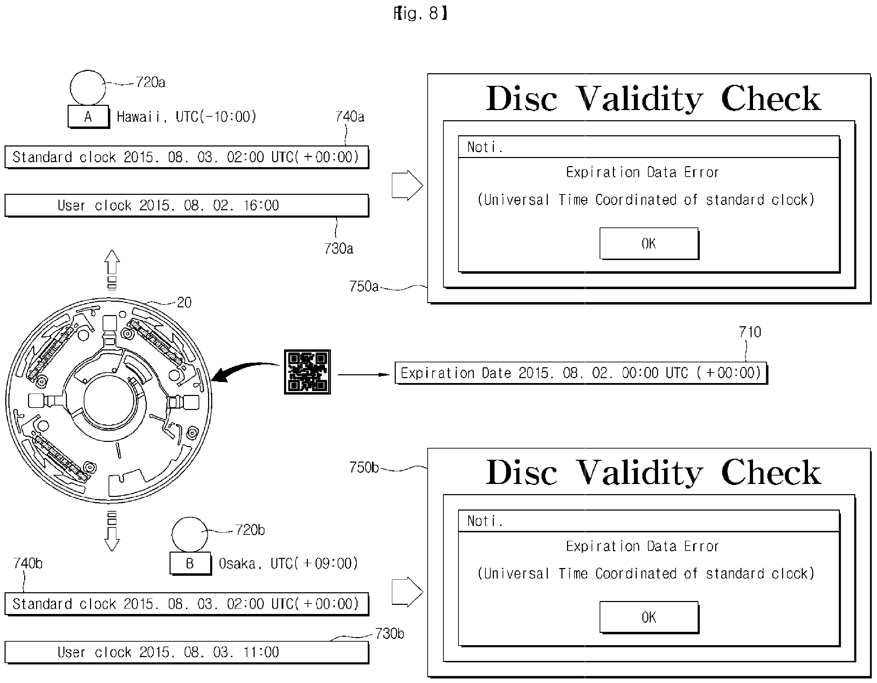

FIG. 8 is a view for describing operation in which the test apparatus according to an embodiment determines the validity date of test medium.

More specifically, FIG. 8 is a view for describing operation in which test apparatuses 100 located in different regions determine whether the validity date of the same test medium 20 has expired.

A user 720a located in Hawaii may set first time information 730a of the test apparatus 100 to local time (UTC (-10:00)), and a user located in Osaka may set first time information 730b of the test apparatus 100 to local time.

In this case, it may be determined that the validity date 710 of the test medium 20 has expired, based on the first time information 730a. However, it may be determined that the validity date 710 of the test medium 20 has not expired, based on the first time information 730b. Accordingly, different determinations on whether the validity date 710 of the test medium 20 has expired based on the first time information 730a and 730b set to local time may be made according to the locations of the test apparatus 100.

Also, a user may set the first time information 730a and 730b of the expired test medium 20 to arbitrary time that is later than the validity date 710 of the test medium 20, for the purpose of misusing the expired test medium 20. If the test apparatus 100 determines expiration of the validity date of the test medium 20 based on the first time information 730a and 730b, the test apparatus 100 cannot prevent such a misuse.

Accordingly, the test apparatus 100 according to an embodiment of the present disclosure may determine whether the validity date of the test medium 20 has expired, based on second time information 740a and 740b set to reference time for determining expiration of the validity date 710, instead of the first time information 730a and 730b which the user can change.

Accordingly, both the test apparatus 100 located in Hawaii and the test apparatus 100 located in Osaka may generate notification messages representing expiration of the validity date 710 of the same test medium 20, regardless of the first time information 730a and 730b. For example, the notification messages may include user interface screens 750a and 750b representing expiration of the validity date 710.

Also, all analyzers of an in-vitro diagnostic apparatus located in Hawaii and an in-vitro diagnostic apparatus located in Osaka will not analyze a test object since the validity date 710 of the test medium 20 has expired.

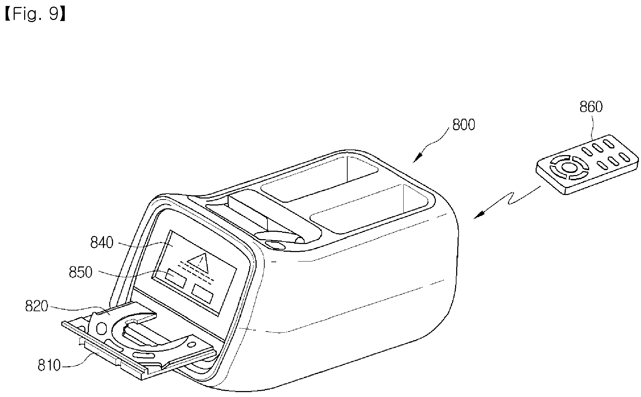

FIG. 9 is a perspective view showing an outer appearance of a test apparatus according to another embodiment of the present disclosure.

Unlike the test apparatus 100 described above with reference to FIGS. 1 to 8, a test apparatus 800 according to another embodiment of the present disclosure may acquire second time information from an external device 860 to thereby prevent a user from arbitrarily manipulating time information of the test apparatus 800 to use the test medium 20 whose validity date has expired. More specifically, since the test apparatus 800 acquires second time information from the external device 860, a manager who is in charge of managing and controlling the second time information can be distinguished from a general user of the test apparatus 800.

If the test apparatus 800 is a blood tester, the test apparatus 800 may be constructed as shown in FIG. 9.

The test apparatus 800 may receive test medium 20 including blood collected from a patient and placed on a test medium inserting surface 820 of a loader 810, analyze the blood included in the test medium 20, and output the results of the analysis through a display 840.

The test medium 20 may be configured to include blood as an object that is to be tested, as described above with reference to FIG. 6. The test medium 20 may be a disc type, a cartridge type, etc.

Also, the test apparatus 800 may start testing blood as a test object according to a predetermined trigger signal informing a test start. For example, the display 840 may be implemented as a touch screen in which a display panel is combined with a touch panel. In this case, a user interface screen which is a menu screen for performing a blood test may be displayed through the display 840. A user may touch a predetermined button 850 included in the menu screen to start a blood test.

Also, the test apparatus 800 may have a portable size, as shown in FIG. 1, and the test apparatus 800 may be installed in transportation means, such as an ambulance or a rescue helicopter, to transport an emergency patient.

The test apparatus 800 may determine validity date of the test medium 20 before starting a blood test.

The test apparatus 800 may acquire second time information from the external device 860, in order to determine the validity date of the test medium 20. As described above with reference to FIGS. 1 to 8, the second time information may be set to reference time that is used to determine the validity date of the test medium 20.

Comparing the test apparatus 100 of FIG. 1 to the test apparatus 800 of FIG. 9, the test apparatus 100 of FIG. 1 may receive current time information from the RTC portion 150 installed therein, whereas the test apparatus 80 of FIG. 9 may receive current time information from the external device 860. A first offset manager may store first time information, and a second offset manager may store second time information, based on the current time information provided from the external device 860.

The test apparatus 800 may communicate with the external device 860 by a wired communication method. More specifically, the test apparatus 800 may communicate with the external device 860 through a data cable connected to the external device 860. For example, the test apparatus 800 may communicate with the external device 860, through at least one of a Universal Serial Bus (USB), a Transfer Controller Protocol/Internet Protocol (TCP/IP), a User Datagram Protocol (UDP), a Universal Asynchronous Receiver Transmitter (UART), a Controller Area Network (CAN), and Institute of Electrical and Electronics Engineers 1394 (IEEE 1394).

The test apparatus 800 may be coupled with the external device 860. For example, the test apparatus 800 may be coupled with the external device 860 which is in the form of a USB drive, without using a USB data cable.

The test apparatus 800 may communicate with the external device 860 by a short-range wireless communication method. For example, the test apparatus 800 may communicate with the external device 860, through Bluetooth, Bluetooth Low Energy, Near Field Communication (NFC), Wireless Local Area Network (WLAN, for example, Wireless-Fidelity (Wi-Fi)), Zigbee, Infrared Data Association (IrDA), Wi-Fi Direct (WFD), Ultra Wideband (UWB), Ant+, or an Infrared or Ultrasonic-waves method.

Also, the test apparatus 800 may communicate with the external device 860 through an external server. For example, the test apparatus 800 may transmit data to the external device 860, or receive data from the external device 860, via a server, through a 3Generation (3G) or 4Gneration (4G) communication network, or Wi-Fi.

FIG. 9 shows the external device 860 which is in the form of a USB drive that can be coupled with a USB socket of the test apparatus 800. The external device 860 may include a RTC portion in which current time information is stored. If the external device 860 is coupled with the USB socket, the test apparatus 800 may acquire current time information from the external device 860.

The test apparatus 100 may acquire validity date information of the test medium 20 from the test medium 20 in order to determine the validity date of the test medium 20. For example, the test apparatus 800 may recognize a QR code of the test medium 20 to acquire validity date information of the test medium 20, as described above with reference to FIG. 6.

The test apparatus 800 may compare the validity date information of the test medium 20 acquired from the test medium 20 to second time information according to the current time information acquired from the external device 860 to determine validity date of the test medium 20.

If the test apparatus 100 determines that the validity date of the test medium 20 has not yet expired, from the result of the comparison between the validity date information of the test medium 20 and the second time information, the test apparatus 800 may analyze a test object based on first time information that can be set to arbitrary time.

If the test apparatus 100 determines that the validity date of the test medium 20 has expired, from the result of the comparison between the validity date information of the test medium 20 and the second time information, the test apparatus 800 may output a notification message informing that the validity date of the test medium 20 has expired, without analyzing the test object.

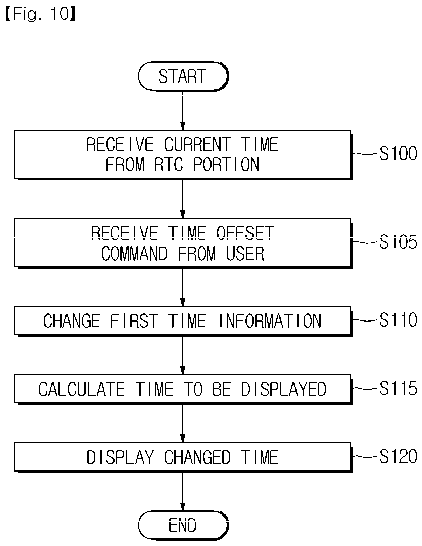

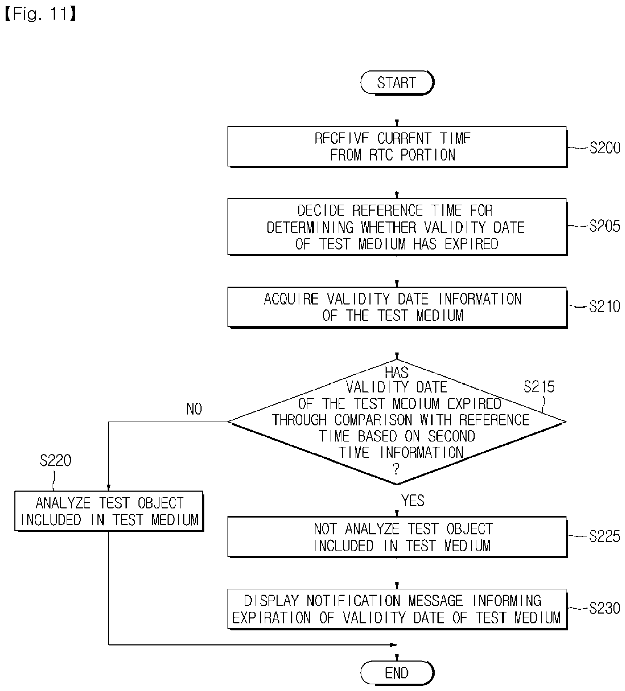

FIG. 10 is a flowchart illustrating a method of controlling a test apparatus to change and display current time, according to an embodiment, and FIG. 11 is a flowchart illustrating a method of controlling a test apparatus to determine whether the validity date of test medium has expired, according to an embodiment.

The test apparatus 100 according to an embodiment of the present disclosure may include the same technical concepts as the operations and configuration of the test apparatus 100 described above with reference to FIGS. 1 to 9. Accordingly, overlapping descriptions as those described above with reference to FIGS. 1 to 9 will be omitted.

Referring to FIG. 10, the RTC portion 150 may provide current time to the first offset manager 151, in operation S100. The RTC portion 150 may receive standard time when the test apparatus 100 was manufactured or when control system of the test apparatus 100 was set initially, store the standard time in an internal register, and output the standard time as current time. The first offset manager 151 may receive information of the current time from the RTC portion 150, and store the information of the current time as first time information.

The user interface 160 may receive a time offset command from a user, in operation S105. That is, since the test apparatus 100 needs to change time to be displayed based on current time information provided from the RTC portion 150, according to a country where the test apparatus 100 is used or according to local time at which the test apparatus 100 is used, the user may input a time offset command for changing time information to be displayed on the test apparatus 100, through the user interface 160.

The time offset command input by the user may be transferred to the first offset manager 151. The first offset manager 151 may change the first time information based on the time offset command, in operation S110, and calculate time to be displayed, in operation S115. That is, as described above, the first offset manager 151 may add a time difference based on the time offset command received from the user to a pre-set initial offset to change the first time information, and calculate time to be displayed according to the changed first time information.

The controller 165 may control the display 140 to display the time changed by the first offset manager 151, in operation S120. Accordingly, the user can use the test apparatus 100 according to time information of a desired country through the time displayed on the display 140.

Referring to FIG. 11, the RTC portion 150 may provide current time to the second offset manager 152, in operation S200. The second offset manager 152 may receive information of the current time from the RTC portion 150, and store the information of the current time as second time information.

The second offset manager 152 may store the current time information provided from the RTC portion 150 as the second time information, and decide reference time for determining whether validity date of the test medium 20 has expired, based on the second time information, in operation S205. The second time information stored in the second offset manager 152 may correspond to reference time that is used to determine whether the validity date of the test medium 20 has expired, and the reference time may be the same time information as that applied to set the validity date of the test medium 20.

The validity date acquirer 170 may be installed in the test medium 20 to recognize a barcode, a QR code, text data, a data matrix, or a recognition pattern including information about the test medium 20, or the validity date acquirer 170 may perform short-range communication, such as NFC and RFID, with the test medium 20 to acquire validity date information of the test medium 20, in operation S210. The validity date information of the test medium 20 acquired by the validity date acquirer 170 may be transferred to the controller 165.

The controller 165 may determine whether the validity date of the test medium 20 has expired, based on the reference time decided by the second offset manager 152 and the validity date information of the test medium 20 acquired by the validity date acquirer 170, in operation S215. More specifically, the controller 165 may compare the validity date information of the test medium 20 acquired by the validity date acquirer 170 to the reference time decided by the second offset manager 152, and if time included in the validity date information of the test medium 20 is earlier than the reference time decided by the second offset manager 152, the controller 165 may determine that the validity date of the test medium 20 has expired, and otherwise, the controller 165 may determine that the validity date of the test medium 20 has not expired.

The controller 165 may control analysis of a test object based on the determination on whether the validity date of the test medium 20 has expired. If the controller 165 determines that the validity date of the test medium 20 has expired, the analyzer 175 may not analyze a test object included in the test medium 20 under the control of the controller 165, in operation S225. If the controller 165 determines that the validity date of the test medium 20 has expired, the display 140 may display a notification message informing expiration of the validity date under the control of the controller 165, in operation S230. The notification message informing expiration of the validity date has been described above with reference to FIGS. 7 and 6, and accordingly, a detailed description thereof will be omitted.

If the controller 165 determines that the validity date of the test medium 20 has not expired, the analyzer 175 may analyze the test object included in the test medium 20 under the control of the controller 165, in operation S220. That is, the analyzer 175 may analyze the test object such as blood to create the result of analysis for determining whether a patient was infected with a predetermined disease.

Meanwhile, the embodiments of the present disclosure as described above can be written as a program that can be executed on a computer, and can be implemented on a general digital computer executing the program using computer-readable recording medium.

The computer-readable medium may be magnetic storage medium (e.g., ROMs, floppy disks, hard disks, etc.), optical reading medium (e.g., CD-ROMs or DVDs), or carrier waves (e.g., transmission through the Internet).

While the present disclosure has been particularly described with reference to exemplary embodiments, it should be understood by those of skilled in the art that various changes in form and details may be made without departing from the spirit and scope of the present disclosure.

## Claims

1. A test apparatus of analyzing a test object included in test medium, comprising:
a Real Time Clock (RTC) portion configured to provide current time;
a user interface configured to receive a time offset command for changing the current time and displaying the changed current time;
a first offset manager configured to reflect the time offset command to the current time provided from the RTC portion to calculate time to be displayed;
a second offset manager configured to decide reference time to be used to determine whether validity date of the test medium has expired, based on the current time provided from the RTC portion;
a validity date acquirer configured to acquire validity date information of the test medium; and
a controller configured to determine whether validity date of the test medium has expired, based on the reference time and the validity date information of the test medium.

2. The test apparatus of claim 1, wherein the first offset manager includes first time information set to arbitrarily change the current time provided from the RTC portion, based on the time offset command.

3. The test apparatus of claim 1, wherein the second offset manager includes second time information set to prevent the current time provided from the RTC portion from being arbitrarily changed.

4. The test apparatus of claim 1, wherein the first offset manager adds a time difference based on the time offset command to a pre-set initial offset to change the current time.

5. The test apparatus of claim 1, further comprising a display configured to display the time calculated by reflecting the time offset command,
wherein the display displays information informing that the validity date of the test medium has expired, when the validity date of the test medium has expired.

6. The test apparatus of claim 1, wherein the validity date acquirer acquires the validity date information of the test medium by recognizing at least one of a barcode, a Quick Response (QR) code, text data, a data matrix, a recognition pattern, Near Field Communication (NFC), and Radio Frequency Identification (RFID) that are installed in the test medium and include information about the test medium.

7. The test apparatus of claim 1, wherein the validity date acquirer comprises a sensor configured to recognize at least one of a barcode, a Quick Response (QR) code, text data, a data matrix, a recognition pattern, Near Field Communication (NFC), and Radio Frequency Identification (RFID).

8. The test apparatus of claim 1, wherein the controller compares the validity date information of the test medium to the reference time to determine whether the validity date of the test medium has expired.

9. The test apparatus of claim 1, further comprising an analyzer configured to analyze the test object included in the test medium if the validity date of the test medium has not expired.

10. The test apparatus of claim 9, wherein the analyzer does not analyze the test object included in the test medium if the validity date of the test medium has expired.

11. The test apparatus of claim 1, further comprising a communication device configured to receive the current time information from an external server.

12. The test apparatus of claim 1, wherein the test medium comprises at least one of a disc and a cartridge.

13. A method of controlling a test apparatus, comprising:
receiving current time;
receiving a time offset command for changing the current time and displaying the changed current time;
reflecting the time offset command to the current time to calculate time to be displayed;
deciding reference time to be used to determine whether validity date of the test medium has expired, based on the current time;
acquiring validity date information of the test medium;
determining whether the validity date of the test medium has expired, based on the reference time and the validity date information of the test medium.

14. The method of claim 13, wherein the reflecting of the time offset command to the current time to calculate the time to be displayed comprises adding a time difference based on the time offset command to a pre-set initial offset to change the current time.

15. The method of claim 13, further comprising displaying the time calculated by reflecting the time offset command.
